(19) **Europäisches Patentamt / European Patent Office / Office européen des brevets**

(11) **EP 2 678 742 B1**

(12) **EUROPEAN PATENT SPECIFICATION**

(45) Date of publication and mention of the grant of the patent:
**29.08.2018 Bulletin 2018/35**

(51) Int Cl.:
*G03C 1/73* (2006.01)   *C07C 233/05* (2006.01)
*B32B 27/10* (2006.01)   *B32B 27/32* (2006.01)
*B32B 29/06* (2006.01)   *B32B 15/12* (2006.01)
*B32B 15/20* (2006.01)   *B32B 15/085* (2006.01)

(21) Application number: **12708378.0**

(22) Date of filing: **24.02.2012**

(86) International application number:
**PCT/GB2012/050424**

(87) International publication number:
**WO 2012/114121 (30.08.2012 Gazette 2012/35)**

(54) **REVERSIBLY ACTIVATABLE DIACETYLENES AND THEIR USE AS COLOUR-FORMERS**

REVERSIBEL AKTIVIERBARE DIACETYLENE UND DEREN VERWENDUNG ALS FARBTRÄGER

DIACÉTYLÈNES ACTIVABLES DE FAÇON RÉVERSIBLE ET LEUR UTILISATION À TITRE DE CHROMOGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.02.2011 GB 201103178**

(43) Date of publication of application:
**01.01.2014 Bulletin 2014/01**

(73) Proprietor: **DataLase Ltd**
**Widnes**
**Cheshire WA8 8FW (GB)**

(72) Inventors:
• **JARVIS, Anthony N**
  **Widnes**
  **Cheshire WA8 8FW (GB)**
• **WYRES, Christopher Anthony**
  **Widnes**
  **Cheshire WA8 8FW (GB)**
• **MULCHIN, Benjamin**
  **Widnes**
  **Cheshire WA8 8FW (GB)**
• **O'ROURKE, Adam**
  **Widnes**
  **Cheshire WA8 8FW (GB)**

(74) Representative: **Gill Jennings & Every LLP**
**The Broadgate Tower**
**20 Primrose Street**
**London EC2A 2ES (GB)**

(56) References cited:
**WO-A1-2010/112940    WO-A2-2009/081385**

Remarks:
The file contains technical information submitted after the application was filed and not included in this specification

**Description**

Field of the Invention

The invention relates to methods of forming a colour on a substrate using reversibly activatable diacetylenes.

Background of the Invention

Packaged foods and drinks, as well as other products, typically have a limited shelf-life from the date of filling and sealing the packaging container to the date of opening the packaging container and/or using the contents. The shelf-life can vary depending on the product *per se* as well as the recommended storage conditions of the filled and sealed container.

Examples of packaging containers are the disposable milk or juice containers (cartons) sold by Tetra Pak. These containers are manufactured from a web or individual blanks of a packaging laminate which is usually paper-based. The packaging laminate typically has thin extruded outer coatings of polyethylene. High-speed filling machines form, fill and seal packaging containers from the packaging laminate.

From a consumer safety perspective, it is important to provide the packaging container with information on shelf-life. This is typically indicated on the packaging container, for example by way of a filling date, a "use by" date, or a "best before" date. Today's packages therefore typically have a printed visible date marking in a colour which is distinguished from other printing (decor) on the package. The date marking can be applied on the package at any stage between filling and being placed on the shelf at a retailer, but the safest way to mark the packages is at the time of filling in the filling machine. Marking is typically done by printing on the outermost surfaces of the packaging containers. The marking can be supplemented with other desired information, for example traceability information relating to the filled product or packaging container, as discussed in WO2010/042001.

There is a risk that marking on packaging containers may be altered or eradicated, either intentionally or unintentionally. For example, markings printed on the outermost surface of a packaging container may be damaged by sliding against the outer surfaces of adjacent packaging containers during transport and handling from filling site to retailer site. Such markings may also be damaged by moisture.

WO2010/042001 discusses laser marking of packaging material (in the form of a web or packaging container) bearing an activatable (laser-sensitive) ink or coating. The packaging material is marked with traceability information. WO2011/035909 also discusses laser marking of packaging material.

It has now been appreciated that the thin extruded outer coatings of packaging laminates give rise to a particular difficulty when they are applied over an activatable ink or coating. The layers are extruded at temperatures up to 325°C. This may cause premature activation of the ink or coating, leading to premature colouration or poor light stability.

Certain diacetylenes are known to be capable of forming colour on exposure to light. 10,12-Pentacosadiynoic acid is an example of such a diacetylene. This compound is colourless in its unreacted state, but on exposure to UV light undergoes a topochemical polymerization reaction to generate a blue-coloured polydiacetylene, which can then be transformed into a red-coloured form by thermal perturbations.

WO2006/018640 discloses the application of diacetylenes, such as 10,12-pentacosadiynoic acid, in multi-colour printing applications in combination with a photoacid or photobase-generating species. Colour-forming diacetylenes, such as 10,12-pentacosadiynoic acid, are typically very reactive, and can undergo the polymerization reaction on exposure to UV light at fluence values as low as 50 mJ.cm$^{-2}$. The consequence of this high reactivity is poor stability to background radiation. Light-sensitive diacetylenes will gradually polymerise and turn blue on storage, sometimes even if stored in the dark. In order to generate colourless coatings with these compounds, it is usually necessary to purify them, via re-crystallisation, prior to use, which is time-consuming and wasteful. Also, any coatings made using these diacetylenes will gradually turn blue on exposure to background radiation. This severely limits the range of applications for such coatings.

Summary of the Invention

It has been observed that certain diacetylenes are capable of undergoing a topochemical polymerization reaction to give coloured diacetylenes only when

they are simultaneously exposed to an additional activating stimulus. Such diacetylenes can be "reversibly activated". It has been found that, in the compounds which are reversibly activatable, the alkyl chain between the diacetylene group and the terminal group has an odd number of carbon atoms, and preferably is a propyl group. Reversible activation is particularly advantageous as the compounds have high environmental stability in coatings or in plastics parts. By "unreactive" is meant "unreactive to polymerisation".

In accordance with a first aspect of the invention, A method of forming a coloured substrate, comprising applying to or incorporating within the substrate a diacetylene compound, and exposing the substrate to (i) a first, activating stimulus that converts the diacetylene compound from an unreactive to a reactive form, and (ii) a second stimulus that causes the reactive form of the diacetylene compound to polymerize and form the coloured substrate, wherein the diacetylene compound reverts to its unreactive form on removal of the activating stimulus and is of formula II:

$$CH_3\text{-}(CH_2)_y\text{-}NH\text{-}CO\text{-}(CH_2)_z\text{-}C{\equiv}C\text{-}C{\equiv}C\text{-}(CH_2)_z\text{-}CO\text{-}NH\text{-}(CH_2)_y\text{-}CH_3 \qquad (II)$$

wherein each y is 9, 11, 13, 15 or 17 and each z is 3 or 5.

In accordance with a second aspect of the invention, a coloured substrate is obtainable using the method according to the first aspect of the invention.

Brief Description of the Drawings

Figure 1 is a schematic cross-sectional view of a packaging laminate according to a preferred embodiment of the invention.
Figure 2 is a schematic view of a method for manufacturing the packaging laminate of Fig. 1.
Figure 3 is a perspective view of a packaging container formed from the packaging laminate of Fig. 1.

Description of Preferred Embodiments

*Diacetylenes*

The reversibly activatable diacetylenes used in this invention are initially synthesized in a form that is unreactive to UV light, and are therefore essentially incapable of undergoing light-induced topochemical polymerization reactions to yield a coloured polydiacetylene. However, when such a diacetylene is exposed to an additional stimulus, e.g. by heating above a certain threshold temperature, it is transformed into a form that is highly reactive and will undergo a UV light-induced topochemical polymerization reaction to yield a coloured polydiacetylene. Such a diacetylene, when allowed to cool to below their activation temperature, is converted back into a form that is unreactive to UV light and will no longer undergo light-induced topochemical polymerisation reaction to yield a coloured polydiacetylene. Consequently, UV light reactivity can be switched on and off merely by the application and removal of the additional stimulus to the diacetylene or substrate comprising it.

Although Y may be any straight-chain, branched or ring group of, say, 1 to 20 carbon atoms, it is preferred that Y is $-(CH_2)_m\text{-}T^1\text{-}Q^1\text{-}Z^1$ wherein m is 0 to 20 and $T^1$, $Q^1$, and $Z^1$ are respectively independently selected from the same groups as T, Q and Z. Preferred compounds of formula I are symmetrical, i.e. Y is $-(CH_2)_n\text{-}T\text{-}Q\text{-}Z$.

The nature of Z (and $Z^1$) is not critical. It may be a straight-chain or branched group, or comprise rings, and has 1 to 20 carbon atoms, and it is optionally substituted. By "optionally substituted" is meant that the Z and $Z^1$ groups may comprise other functional groups known in organic chemistry such as ether groups (-O-) or hydroxyl groups (OH).

Z is preferably a straight-chain alkyl group of 12 to 18 C atoms. n is generally in the range 1-21 and is preferably 3.

Similarly, the nature of E (if present) is not critical. If alkyl, it may include unsaturation.

Preferably, in the compounds of the invention, T = CO. Q is preferably NH. Preferred reversibly activatable diacetylenes used in this invention are diacetylene carboxylic acids; particularly preferred are those that have been further derivatised into amides using primary amines that comprise a hydrocarbon alkyl chain of a particular length. The most preferred diacetylenes are symmetrical bis-alkylamides based on 5,7-dodecadiynedioic acid, 7,9-hexadecadiynedioic acid, 3,5-octadiynedioic acid, 9,11-eicosadiynedioic acid and 11,13-tetracosadiynedioic acid. Such amines are of formula II, i.e. 5,7-dodecadiynedioic acid bis(decylamide), 5,7-dodecadiynedioic acid bis(dodecylamide), 5,7-dodecadiynedioic acid bis(tetradecylamide), 5,7-hexadecadiynedioic acid bis(hexadecylamide), 5,7-dodecadiynedioic acid bis(octadecylamide), 7,9-hexadecadiynedioic acid bis(decylamide), 7,9-hexadecadiynedioic acid bis(dodecylamide), 7,9-hexadecadiynedioic acid bis(tetradecylamide), 7,9-hexadecadiynedioic acid bis(hexadecylamide) and 7,9-hexadecadiynedioic acid bis(octadecylamide).

In a preferred embodiment, T = CO, Q = NH and Z = a straight-chain alkyl group of 12 to 18 C atoms. Most preferably, in a symmetrical structure, x = 3, T' = CO, Q' = NH and Z' is a straight-chain alkyl group of 12 to 18 C atoms.

Other diacetylenes that may be used in the present invention include, but are not limited to, 3,5-octadecadiynoic acid, 5,7-eicosadiynoic acid, 7,9-docosadiynoic acid, 9,11-tetracosadiynoic acid, 11,13-hexaicosadiynoic acid, 5,7-dodecadiynoic acid, 5,7-eicosadiyn-1-ol, 5,7-hexadecadiynoic acid, 5,7-octadecadiynoic acid and 5,7-tetradecadiynoic acid and derivatives thereof. Particularly preferred derivatives are amides.

Where the reversibly activatable diacetylene compound is a dicarboxylic acid or a derivative thereof, it can be either symmetrical or unsymmetrical. Where the diacetylene dicarboxylic acid is unsymmetrical, it can be with respect to the number of $CH_2$ units between the diacetylene group and the carboxylic acid group and/or with respect to the derivatisation or the carboxylic acid group.

The most preferred carboxylic acid derivatives are amides, ester and thioesters. These can readily be made by reacting a diacetylene carboxylic acid with a chlorinating agent such as oxalyl chloride and then reacting the diacetylene acid chloride with a nucleophilic compound such as an amine, alcohol or thiol. Amides (-CONR-) are particularly preferred still, where R = H or alkyl group.

A particularly preferred series of amides are those derived from primary amines to give the -CONH- amide group. A primary amine is a compound having the formula $R-NH_2$, where R is H or any group known in organic chemistry comprising at least one carbon atom.

Amides of the type -CONHR can readily be made by reacting a diacetylene carboxylic acid with a chlorinating agent such as oxalyl chloride and then reacting the diacetylene acid chloride with a primary amine in the presence of a base. Particularly, preferred primary amine, are those that comprise saturated, aliphatic hydrocarbon chains, represented by the formula $C_mH_{2m+1}$ where m is an integer $\leq 50$, more preferably still $\leq 20$.

The saturated, aliphatic hydrocarbon groups can be either straight chained, branched or rings. Straight chains are particularly preferred. Examples of saturated, aliphatic hydrocarbon straight-chain primary amines comprising 1 to 20 carbon atoms include methylamine, ethylamine, propylamine, butylamine, pentylamine, hexylamine, heptylamine, octylamine, nonylamine, decylamine, undecylamine, dodecylamine, tridecylamine, tetradecylamine, pentadecylamine, hexadecylamine, heptadecylamine, octadecylamine, nonadecylamine and eicosamine.

Examples of preferred branched, aliphatic primary amines include 2-ethylhexylamine and isopentylamine. The group R in the primary amine can also comprise other functional groups known in organic chemistry such as ether (-O-) or hydroxyl groups (OH).

Where the reversibly activatable diacetylene carboxylic acid compound comprises more than one carboxylic acid group, any number of them can be derivatised into an alkylamide. Other saturated amines that can be used to create reversibly activatable diacetylene carboxylic acid amides that form part of the present invention also include alcohol amines such as ethanolamine, propanolamine, butanolamine, pentanolamine, hexanolamine, heptanolamine, octanolamine, nonanolamine, decanolamine, undecanolamine and dodecanolamine. The alcohol amine compound can comprise more than one OH group, such as 3-amino-1,2-propanediol, 2-amino-2-(hydroxymethyl)-1,3-propanediol and bis-homotris. Also included are ethoxylated amines such as amino-PEGs and 2,2'-(ethylenedioxy)bis(ethylamine).

Another series of particularly preferred primary amines that can be used to create the activatable diacetylenes of the present invention are amino-carboxylic acids. These are compounds that comprise both an amine group and a carboxylic acid group. Examples include alpha-amino acids found in nature, such as glycine, proline, cysteine, selenocysteine, alanine, isoleucine, leucine, methionine, phenylalanine, tryptophan, valine, serine, threonine, asparagine, glutamine, glutamide, glutamic acid, aspartic acid, lysine, histidine and arginine. Other amino-carboxylic acid compounds are 4-aminobutanoic acid, 5-aminopentanoic acid, 6-aminohexanoic acid, 7-aminoheptanoic acid, 8-aminooctanoic acid, 9-aminononanoic acid, 10-aminodecanoic acid, 11-aminoundecanoic acid and 12-aminododecanoic acid. Also included are derivatives of the amino acids and amino carboxylic acid compounds listed above.

These reversibly activatable diacetylene compounds can be made by reacting a diacetylene carboxylic acid compound with an amino acid or amino carboxylic acid compound as described above and then derivatising the carboxylic acid group that was originally part of the amino acid or amino carboxylic acid compound. However, the preferred way to make these compounds is to take the amino-acid or amino-carboxylic acid compound, protect its amine group, using any known protecting group in organic chemistry, derivatise the carboxylic acid groups, de-protect the amine group using any known system in organic chemistry and then react the amine with the diacetylene carboxylic acid compound to yield the final product.

The most preferred type of activatable poly-ynes are diacetylene-amides and diacetylene-bis-amides made by reacting a diacetylene mono or dicarboxylic acid compound with a primary aliphatic hydrocarbon amine, particularly preferred are straight chained primary aliphatic hydrocarbon amines.

*Activation Stimuli*

Activation stimuli that cause the diacetylene to convert from its inactive into its active form include heat, light, pressure, chemicals, solvents, pH changes, biological entities and electrical discharge. Indeed, the stimulus can be any that allows the diacetylene to flip between active and inactive forms.

The most preferred stimulus is heat. The heat can be provided by a hot air source, direct thermal contact, or NIR radiation in the wavelength range 700 nm to 2500 nm. If NIR radiation is used as the source of heat, it is preferred that the system also comprises an NIR-absorber that absorbs the NIR light and converts it into conductive heat. Preferably, the NIR-absorber has an absorbance profile that matches the emission wavelength of the laser.

The light can be of any wavelength in the range 100 nm to 20,000 nm. The light can be non-coherent, coherent, broadband or monochromatic light. A particularly preferred type of light source is that provided by a laser. The laser can be a UV, visible, NIR or mid-infrared $CO_2$-type laser.

Using heat allows the temperature of the sample to be raised to its activation threshold level or above. Exposing this hot form to UV light converts it into its coloured polydiacetylene form. Allowing the system to cool to below its activation threshold temperature renders inactive any poly-yne that had not been polymerized into its coloured form.

*Other effects*

Without being constrained by theory, the colour-forming mechanism of a diacetylene is a topochemical polymerization to yield a polydiacetylene comprising a network of conjugated alternate double and triple bonds: -C=C-C≡C-C=C-

It is also known that polydiacetylenes have electrical conductivity properties. The activatable and reversibly activatable diacetylenes used in the present invention can also be used to create light-printable electronics. It is especially preferred that the diacetylenes are applied onto thin flexible substrates. Such a substrate could be used in the manufacture of circuit boards, electronic displays, photovoltaics, printed sensors, smart packaging and textiles.

*Coatings*

The reversibly activatable diacetylene and NIR-absorbing agent (if present) are typically applied to a substrate via an ink formulation. The ink formulation can be aqueous or non-aqueous. This can be an ink formulation that comprises both the colour-forming compound and NIR-absorbing agent (if present). Alternatively, they can be applied separately, a first coating layer comprising one of the two species beneath an upper layer comprising the other. The ink formulation(s) can also comprise other additive(s) known in the art of printing, such as binders which are typically polymers and include acrylic polymers, styrene polymers and hydrogenated products thereof, vinyl polymers and derivatives thereof, polyolefins and hydrogenated and epoxidised products thereof, aldehyde polymers, epoxide polymers, polyamides, polyesters, polyurethanes, sulphone-based polymers and natural polymers and derivatives thereof such as cellulose-based binders. The binder can also be a mixture of polymeric binders and a core-shell system. It can also be a mixture of liquid monomers and a suitable photo-initiator that forms one of the above polymeric binders under UV irradiation after coating. Examples of suitable binder systems include the Glascol and Joncryl products supplied by BASF, the Paranol products supplied by ParaChem, the Witcobond products supplied by Baxenden Chemicals, the Texicryl products supplied by Scott-Bader and the Neo products supplied by DSM NeoResins+. Other components of the ink formulation(s) may include solvents, surfactants, stabilizers, thickeners, waxes, opacifying agents, whitening agents such as $TiO_2$, anti-foam agents, bases, biocides, colourants, rheology modifiers, light absorbers, anti-oxidants, light stabilizing agents, wetting agents, colorants, smoke-suppressants and taggants.

It is also possible for the activatable light-reactive colour-change compound and the NIR-absorbing agent (if present) to be applied to a substrate without the use of a coating. They can be directly embedded/incorporated in the substrate, and are typically added to the substrate during its manufacture. It is also possible for one of the components to be embedded/incorporated into the substrate but the other applied via a coating.

*NIR light-absorbing agent*

NIR light-absorbing agents are compounds that absorb light in the wavelength range 700 to 2500 nm. Specific examples of the type of compound that can be used in the present invention include:

    i. Organic NIR-absorbing agents
    ii. NIR-absorbing 'conductive' polymers
    iii. Inorganic NIR-absorbing agents
    iv. Non-stoichiometric inorganic absorbing agents.

Particularly preferred NIR-absorbing agents are those that have essentially no absorbance in the visible region of the spectrum (400 to 700 nm) and thus give rise to coatings that appear visibly colourless.

Organic NIR-absorbing agents are known as NIR dyes/pigments. Examples include metallo-porphyrins, metallo-thiolenes and polythiolenes, metallo-phthalocyanines, aza-variants of these, annellated variants of these, pyrylium salts, squaryliums, croconiums, amminiums, diimoniums, cyanines and indolenine cyanines.

Examples of organic compounds that can be used in the present invention are disclosed in US6911262, and are given in Developments in the Chemistry and Technology of Organic dyes, J Griffiths (ed), Oxford: Blackwell Scientific, 1984, and Infrared Absorbing Dyes, M Matsuoka (ed), New York: Plenum Press, 1990. Further examples of the NIR dyes or pigments of the present invention can be found in the Epolight™ series supplied by Epolin, Newark, NJ, USA; the ADS series supplied by American Dye Source Inc, Quebec, Canada; the SDA and SDB series supplied by HW Sands, Jupiter, FL, USA; the Lumogen™ series supplied by BASF, Germany, particularly Lumogen™ IR765, IR788 and IR1050; and the Pro-Jet™ series of dyes supplied by FujiFilm Imaging Colorants, Blackley, Manchester, UK, particularly Pro-Jet™ 830NP, 900NP, 825LDI and 830LDI. Further examples can be found in the products sold by HW Sands and Few Chemicals GmbH. Further examples are disclosed in WO2008/050153.

Examples of NIR-absorbing 'conductive' polymers include PEDOT such as, the Clevios products supplied by HC Starck, and the Orgacon products supplied by Agfa. Further examples are disclosed in WO2005/12442.

Examples of inorganic NIR-absorbing agents include copper (II) salts. Copper (II) hydroxyl phosphate (CHP) is particularly preferred. Further examples are disclosed in WO2005/068207.

Examples of non-stoichiometric inorganic absorbing agents include reduced indium tin oxide, reduced antimony tin oxide, reduced titanium nitrate and reduced zinc oxide. Further examples are disclosed in WO2005/095516. Reduced indium tin oxide is particularly preferred in combination with a 1,400 nm to 2500 nm laser.

It is particularly preferred that the absorption profile of the NIR-absorbing agent approximately matches the emission wavelength of the NIR light source employed.

NIR-absorbing agents are preferred; however, the invention is not limited to these. Other light-absorbing agents that can be used include UV (200 to 400 nm), visible (400 to 700 nm) and mid-infrared (-10.6 microns) light-absorbing agents. Examples include dyes/pigments, UV absorbers and Iriodin type agents.

*Other Colour-Change Chemistries*

The coatings and substrates of the present invention can also comprise other 'non-activatable' colour-change chemistries that are directly responsive to light, in particular laser light. Examples include metal oxyanions, particularly molybdates and borates, more particularly octamolybdates and metaborates with ammonium octamolybdate and sodium metaborate being the most preferred.

The coatings and substrates can also comprise charrable agents such as polysaccharides, carbohydrates and sugars, including cellulose and derivatives thereof, glucose, saccharose, sucrose, maltodextrin, lactose, starch, dextrose, polydextrose and gums.

The coatings and substrates can also comprise metal salts such as base-generating agents such as sodium bicarbonate and sodium carbonate.

The coatings and substrates can also comprise colour-forming agents such as leuco dyes and charge-transfer agents. These can used in combination with photo or thermal acid or base-generating agents. Particularly preferred photoacid generating agents include "onium types" such as sulphonium or iodonium salts. Further examples of photoacid-generating agents include amine adducts of aromatic sulphonic acids such as amine adducts of dinonylnaphthalenedisulphonic acid and tosylates. Other acid-generating 'onium' compounds include ammonium compounds, amines, sulphate, phosphats, hydrogen phosphates, dihydrogen phosphates and borates.

Further examples of the chemistries that can be used in combination with the present invention are disclosed in WO2006/129086, WO2007/045912, WO2002/068205, WO2006/129078, WO2004/043704, WO2002/074548, WO2007/063339, WO2006/051309 and WO2009/010393.

*Substrates*

The substrate can be any substrate known in the art of printing. Examples include paper, paperboard, cardboard, corrugate, glass, textiles including knitted woven and non-woven fabrics, yarns, fibres, carpets, metal, foils, wood, leather, plastics films, ridged plastics parts, cellulose films, foodstuffs and pharmaceutical preparations. If paper is used, it can be clay-coated and laminated. The substrate may be a data carrier such as a CD or a DVD. The activatable diacetylene can be applied to the substrate using an ink or surface coating formulation, or it can be embedded directly into the substrate such as paper or paperboard by being added during the sizing stage, or extruded into a plastics film. The substrate can be laminated or remain unlaminated.

The substrate comprising the reversibly activatable diacetylenes of the present invention can be used in the manufacture of printed items, examples include primary and secondary packaging, newspapers, magazines, leaflets, pamphlets and books, posters, labels in combination with an adhesive backing, security documents such as banknotes, cheques, currency, tickets, passports and licences. It can be used in desk-top/home printing and commercial wide-web printing applications. The substrate comprising the reversibly activatable diacetylenes can also be used to make containers suitable for use in sterilized food packaging applications. The substrate can also be any used in printed electronics applications such as a circuit board manufacture. The substrate can be used to display human-readable and/or machine-readable information, such as text, graphics or barcodes.

*Plastics Colouration*

The reversibly activatable poly-ynes of the present invention are also particularly suitable for use in plastics colouration. They can be used for bulk colouration, or for printing images, patterns, devices, machine-readable codes and text directly on to the plastics part using either a laser-scanning system, an array system or a lamp/mask arrangement. The activatable poly-yne can be delivered to the plastics via a solid or liquid masterbatch system. Examples of suitable plastics include acrylonitrile-butadiene-styrene (ABS), arylic (PMMA), celluloid, cellulose acetate, cycloolefin copolymer (COC), ethylene-vinyl acetate (EVA), ethylene-vinyl alcohol (EVOH), fluoroplastics (PTFE, and also FEP, PFA, CTFE, ECTFE, ETFE), ionomers, Kydex (a trade-marked acrylic/PVC alloy), liquid crystal polymer (LCP), polyacetal (POM or acetal), polyacrylates (acrylic), polyacrylonitrile (PAN or acrylonitrile), polyamide (PA or nylon), polyamide-imide (PAI), polyaryl ether ketone (PAEK or ketone), polybutadiene (PBD), polybutylene (PB), polybutylene terephthalate (PBT), polycaprolactone (PCL), polychlorotrifluoroethylene (PCTFE), polyethylene terephthalate (PET), polycyclohexylene dimethylene terephthalate (PCT), polycarbonate (PC), polyhydroxyalkanoates (PHAs), polyketone (PK), polyester, polyethylene (PE) of low and high density, polyether ether ketone (PEEK), polyetherketoneketone (PEKK), polyetherimide (PEI), polyethersulfone (PES), polysulfone, polyethylene chlorinates (PEC), polyimide (PI), polylactic acid (PLA), polymethylpentene (PMP), polyphenylene oxide (PPO), polyphenylene sulfide (PPS), polyphthalamide (PPA), polypropylene (PP), polystyrene (PS), polysulfone (PSU), polytrimethylene terephthalate (PTT), polyurethane (PU), polyvinyl acetate (PVA), polyvinyl chloride (PVC), polyvinylidene chloride (PVDC), and styrene-acrylonitrile (SAN). The plastics can also comprise other additives known in the plastics processing industry such as colourants, clarifiers, foaming agents, nucleating agents, toners, light-barriers, opacifying agents such as $TiO_2$, process aids, slip agents, oxygen-scavengers, barriers such as $CO_2$ and $O_2$ barriers, reheat agents, anti-acetaldehyde agents, UV absorbers, HALS and light-stabilization agents.

A plastic comprising the activatable or reversibly activatable poly-yne compound can be used to make any plastics part, examples including ridged plastics packaging such as preforms, containers, bottles and closures, or melt-spun fibres that are used in the manufacture of, for example, non-woven fabrics for use in pads, nappies and feminine hygiene products.

The reversibly activatable diacetylenes can be applied to the plastics as a neat powder, or via a solid or a liquid masterbatch. Suitable examples of solid and liquid masterbatch products and dosing systems are supplied by Americhem, Colormatrix and Polyone, all of OH, USA.

*Light Sources*

If the initial reversible activation is to be caused by a light source, that is preferably one that can be used to heat the initially unreactive poly-yne to or above its activation threshold temperature. It can be light in the wavelength range 200 nm to 25 $\mu$m. More preferably, it is near-infrared light in the wavelength range 700 nm to 2,500 nm, and even more preferably approximately corresponds with the absorbance profile of the NIR light absorbing agent used. The light can be broadband or mono-chromatic, non-coherent or laser radiation. Preferably the light is NIR laser radiation. The laser can be a pulse or continuous wave laser, a fibre laser or a diode laser, or an array of diodes. A $CO_2$ laser operating with an approximate wavelength of 10.6 $\mu$m is preferred.

The light used to initiate the colour change reaction of the previously activated colour-forming compound can be in the wavelength range 200 nm to 25 $\mu$m. More preferably, it is UV light in the wavelength range 200 to 400 nm, or short wavelength visible light in the range 400 to 450 nm. The light can be broadband or mono-chromatic, non-coherent or laser radiation. The light can be non-coherent light as supplied by a lamp and is used merely to flood the whole substrate surface with light. Examples of UV light sources that can be used include germicidal lamps and mercury arc lamps. Alternatively, a UV laser or UV diode light source can be used, particularly where more precise placement of light is required. A lamp/mask arrangement can alternatively be used. A $CO_2$ laser operating with an approximate wavelength of 10.6 $\mu$m is preferred, particularly if chemistries responsive to $CO_2$ laser light are also present.

Where a laser system is employed, it can be a pulsed or continuous wave laser. The light beam can be steered using a mirror-based galvanometer type system or emitted from an array of light sources. The colour-change reaction can alternatively be induced by heat, which can be contact or non-contact heat.

The present invention also includes the use of photoacid generators, such as those disclosed in WO2006/018640, to sensitise the diacetylene alkylamide to light of longer wavelength than its intrinsic absorption, once it has been activated.

The reversibly activated poly-ynes of the present invention can be coloured by the sequential application of heat to raise the temperature of the poly-yne to greater than its activation threshold temperature, followed by UV light exposure to induce the topochemical polymerization reaction to yield the colour form. However, the heat and UV light can also be applied simultaneously. This can be done through a NIR laser in combination with an UV light source such as UV diodes, a UV laser or a UV lamp.

*Packaging Laminate*

A typical packaging laminate has a base or substrate layer, which can be a paper or paperboard layer or a layer of a plastic. Preferably, said base layer is a layer of paperboard which is renewable and therefore environmentally friendly and which provides required mechanical strength to the packing container to withstand outer stresses during normal transport and handling of the container. In a preferred embodiment, the base layer is a clay-coated paperboard. Clay coating improves printability of the packaging laminate. Clay coating may take place on one side (the side which will face the outside of the packaging container) only or on both sides.

In order to protect the base layer from penetration of liquid and humidity, which otherwise would make the layer soggy and unsuitable for forming containers, the packaging laminate usually comprises outer liquid-tight layers of plastics on each side of the paper or paperboard layer as mentioned above. Preferred examples of such outer layers are films or coatings of polyolefins (especially polyethylene, polypropylene) and polyester. Most preferably, said outer films or coatings are low density polyethylene (LDPE) coatings. The outer layers are preferably colourless and transparent. The outer coating which will face the inside of the packaging container may comprise mLLDPE (linear low density polyethylene produced using a metallocene catalyst).

Where the packing container is intended to be filled with an oxygen-sensitive product (such as juice, wine or edible oil), the packaging laminate is typically supplemented with at least one additional layer having desired barrier properties against gases, in particular oxygen. A preferred gas barrier for use in such a packaging laminate is an aluminium foil or a metallised film. Other useful gas-barrier layers are film layers of barrier polymers, such as ethylene vinyl alcohol copolymer (EVOH) and polyamide (PA), which may be used separately or in combination with an aluminium foil or metallised film. Preferably, the barrier layer is an aluminum foil which enables a high-speed effective heat sealing through inductive heating of the laminate during manufacture of packing containers. The oxygen-barrier layer, where present, is applied between the base layer and one of said outer liquid tight layers. Preferably, it is applied on the side of the base layer which will face the inside of the packaging container.

A packaging laminate of the invention includes a diacetylene compound of general formula (I) as described above, preferably in the form of an ink. The diacetylene compound is preferably positioned between the base layer and the outer layer which will face the outside of the packaging container. In this way, the coloured mark formed from the diacetylene compound is protected by the outer layer but is visible to the consumer. As an alternative, however, the diacetylene compound may be included in the outer layer or another layer, or may be positioned between the base layer and the outer layer which will face the inside of the packaging container.

The diacetylene compound may cover part or all of the area of the packaging laminate, but preferably covers only part of the area of the packaging laminate, suitably in the form of one or more patches.

Preferably, the diacetylene compound is colourless/white and transparent such that it is at least substantially invisible. This means that it can be applied without the need to take the package decor into account. If the diacetylene compound is not colourless and transparent, an empty area on the packaging laminate can be used to avoid interference with the package decor. Preferably, package decor does not overlap with areas where the diacetylene compound is to be activated.

*Packaging container*

A packaging container is preferably for food or drink, for example milk, other liquid dairy products, and juices. It may be brick-shaped. The packaging container is preferably filled and sealed.

The packaging container is provided with a coloured mark which is visible from the outside of the container. "Coloured" means that there is a visible contrast between the mark and unexposed diacetylene compound. The mark may be monochrome (e.g. black or dark blue-magenta) or multi-coloured. The mark is preferably in the form of a desired pattern, and in particular is preferably used to display human-readable information such as filling dates, "use by" dates and "best before" dates. Other traceability information or consumer-directed information as discussed in WO2010/042001 may also be displayed in the coloured mark (for example, in small batch runs product information may be displayed in this way to provide flexibility). The mark may be based on non-visible information carried in the packaging laminate, for example via magnetic marks of the type discussed in WO2010/042001.

*Method of making laminate*

A packaging laminate is preferably produced in the form of a web, but may be in the form of sheets. When in the form of a web, form-fill-seal technology is commonly used.

The diacetylene compound, suitably in the form of an ink, is preferably applied to the base layer. This is preferably done by printing, for example using a rotary printing technique. Preferably, package decor is printed in the same step. Alternatively, the diacetylene compound can be applied using a surface coating formulation, or it can be embedded directly into the base layer by being added during the sizing stage. As a further alternative, the diacetylene compound could be applied to the outer layer or another layer, or, as mentioned above, could be included in one of the layers itself.

Optionally, the base layer is provided with an appropriate design of weakening lines (so-called crease lines) so as to facilitate folding of the packaging laminate in the filling machine. The creasing operation may be performed in the same station as the printing operation. Conventionally, the creasing operation is performed as close as possible to the printing operation so as to achieve best possible alignment between printing (decor and diacetylene compound) and the pattern of crease lines.

Optionally, a gas barrier foil or film as described above is laminated to the base layer, preferably on the non-printed side of the base layer. The gas barrier layer may be adhered to the laminate layers by means of intermediate adhesive or bonding layers if needed.

The outer liquid-tight coatings or films are applied to each side of the laminate, for example by coating or lamination.

The laminate is typically exposed to a mechanical finishing (slitting and trimming) operation and rolled onto a roller.

*Method of making packaging container*

The packaging container may be produced from a web of packaging laminate or individual blanks.

From a web, the filling machine first forms a tube by permanently joining the longitudinal edges of the web to each other in an overlap joint. The tube is filled with its relevant food, e.g. juice, and repeatedly transversely sealed by compressing and heat-sealing the tube below the product level to form continuous individual package units which are separated from each other through cutting. Heat-sealing may rely on inductive heating of an aluminium foil layer as mentioned above, or UV light or heat may be used. Following a final folding and sealing operation, the packaging containers are ready for further transport and handling.

Forming the coloured mark from the diacetylene compound is achieved using first and second stimuli as described in more detail elsewhere in this specification. These stimuli are preferably applied in the filling machine, and more preferably during or close to (e.g. immediately after) the filling stage. Suitable stimuli include light sources such as lasers, and heat guns.

Preferably, the first stimulus is NIR radiation. Suitable NIR lasers are described in WO2010/042001. YAG and diode lasers are preferred because of their small size which makes them suitable for use in filling machines. $CO_2$ lasers may be less preferred because they are larger. A preferred fluence range is 2.5-3.5 $J/cm^2$, for example about 3 $J/cm^2$ or about 4 $J/cm^2$. Usually the presence of an outer coating over the diacetylene compound causes fluence requirements to increase, as the outer coating acts as a thermal sink and draws heat away from the colour change reaction.

Preferably, the second stimulus is UV light. It may be applied using a UV lamp. A preferred fluence is about 0.5 $J/cm^2$.

Preferably, at least one of the first and second stimuli is applied selectively, i.e. not to the whole area of the diacetylene compound. This selective exposure is used to form the desired coloured mark. In a preferred embodiment, the first stimulus is applied selectively and the second stimulus is applied generally.

The shape of the coloured mark may be controlled, for example by means of a projected picture (e.g. via a lamp/mask arrangement) or by direction of a beam of light, as discussed in more detail in WO2010/042001 and WO2011/035909 and elsewhere in this specification.

The packaging laminate aspect of the invention will now be disclosed in greater detail with reference to the accompanying drawings.

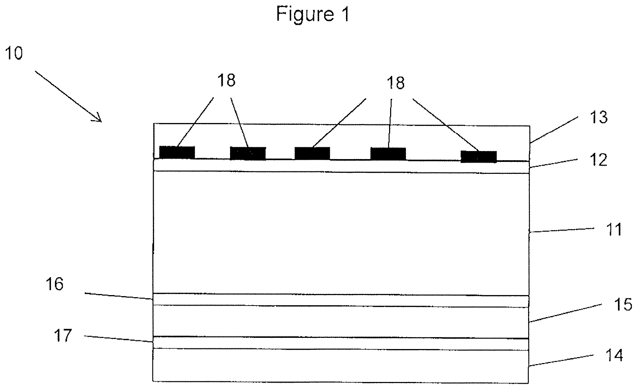

Fig. 1 shows a packaging laminate 10 that comprises a base or substrate layer 11 of paperboard having on its upper surface a clay coating 12. The packaging laminate 10 is provided with an outer LDPE liquid-tight layer 13 (upper surface) and an outer mLLDPE liquid-tight layer 14 (lower surface). These layers are transparent and colourless.

The packaging laminate 10 also includes an oxygen gas barrier layer 15 between the base layer 11 and outer film 14. The gas barrier layer 15 is adhered to the laminate layers 11 and 14 by means of intermediate adhesive or bonding layers 16 and 17, respectively.

The base layer 11 has an inactivated but activatable invisible (transparent and colourless) marking 18 (shown as shaded areas) applied directly in the clay coated surface 12 of the base layer 11. The marking 18 is made of a diacetylene compound-containing ink such as that of Example 2 (below).

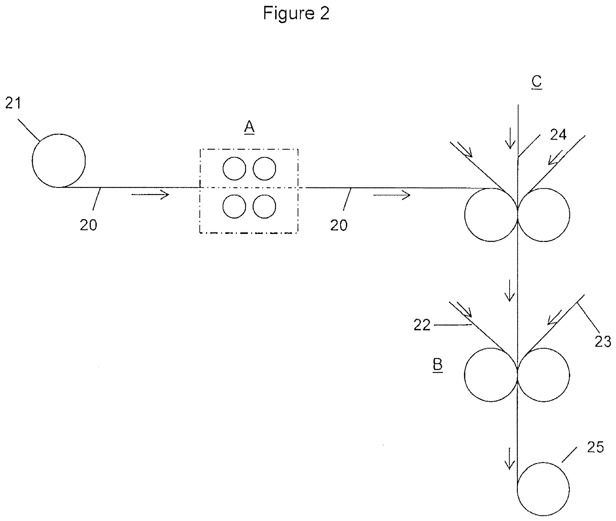

To form the packaging laminate, a web 20 of clay coated paperboard (corresponding to base layer 11 and clay coating 12 of the packaging laminate 10 of Fig. 1) is unrolled from a roller 21 and is passed through a printing station at A (Fig. 2). In this station, the web 20 is provided in some areas with the marking 18 through a rotary printing technique. In the same printing station A, the web is provided with a desired decor which is applied on the same surface, but on areas not occupied by invisible marking 18.

The printed web 20 is also provided with crease lines at station A The printed and creased web 20 is provided with aluminium foil as a gas barrier layer 24 in a lamination station C. The web 20 is then provided on each side with extrusion coated outer liquid-tight coatings 22 and 23 of LDPE at a coating station B (which contains two extruders). Finally, the web 20 is exposed to a mechanical finishing (slitting and trimming) operation and rolled on roller 25. The markings 18 remain invisible at this stage.

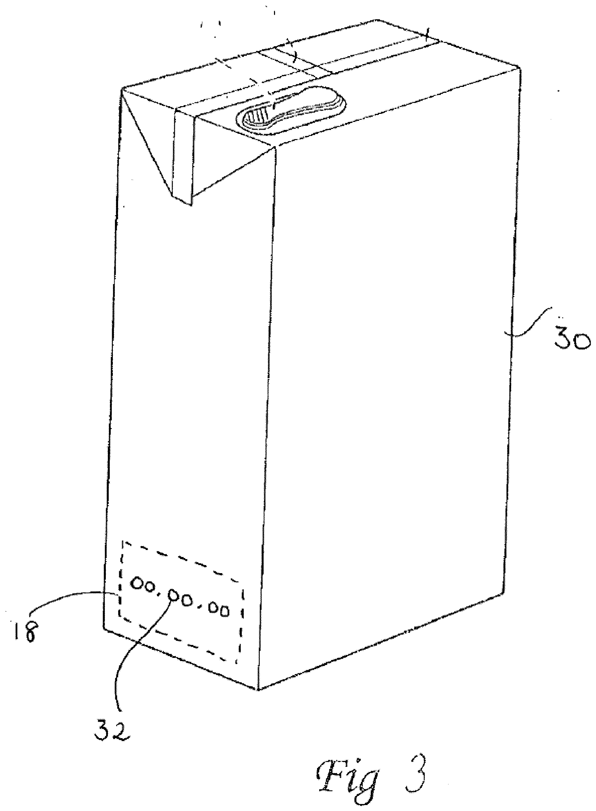

The web 20 (substrate) is introduced to a high-speed filling machine where a form-fill-seal operation takes place to produce filled packaging containers 30 (Fig. 3). After the filling and sealing stages, the invisible markings 18 are treated with first and second stimuli as follows.

Firstly, the invisible markings 18 are selectively heated using a NIR laser (first, activating stimulus) in a pattern corresponding to the desired coloured mark 32 according to signals from a processing unit. The processing unit ensures correct alignment in the filling machine, which operates at high speed with small tolerances. This causes the markings to become reversibly activated. Secondly, UV light (second stimulus) is generally applied using a UV lamp.

In this way, the invisible markings 18 are selectively coloured to produce a desired coloured mark 32. The mark typically represents one or more of filling date, use-by date and best before date (human readable information).

This preferred embodiment of the invention has various advantages:

- The invisible markings 18 and final coloured mark 32 are positioned under the transparent and colourless outer layer 13. This means that they are resistant to tampering and damage, but the final coloured mark can be seen by consumers. Any tampering is likely to be evident. The markings are also protected from moisture.
- As described in more detail above, the invisible markings 18 need to be subjected to a first activating stimulus and a second stimulus to be permanently coloured. This means that premature activation (colouring or poor light stability) of the invisible markings 18 (for example when they are subjected to heat during extrusion lamination of the outer films) is unlikely to occur.
- Formation of the final coloured mark 32 is achieved by selective application of NIR laser light. No contact is needed between the light source and the package, by contrast with a printer which is likely to be subjected to much more wear.
- It is believed that the ink used is suitable for use in indirect food packaging applications.
- The ink used is stable and compatible with the printing process used for package decor.

The following Examples illustrate the invention.

**Example 1**

5,7-Dodecadiynedioic Acid Bis(octadecylamide)

5-Hexynoic acid was Glazer-Eglinton-Hay coupled in the presence of copper (I) bromide and oxygen, into 5,7-dodecadiyndioic acid. The 5,7-dodecadiyndioic acid was converted into its acid chloride by treatment with excess oxalyl chloride and a catalytic amount of dimethylformamide. The 5,7-dodecadiyndioic acid chloride that formed was then reacted with excess octadecylamine in the presence of an equivalent amount of triethylamine (a tertiary base proton scavenger).

The resultant 5,7-dodecadiynedioic acid bis(octadecylamide) was found to have a melting point of 180°C. It appeared to undergo a first phase transition at around 135 to 140°C.

**Example 2**

An ink formulation was prepared from:

| Raw Material | pbw |
|---|---|
| Joncryl LMV7085 - aqueous styrene acrylic binder | 37.5 |
| Water | 10 |
| Dispelair CF49 - mineral oil anti-foam | 0.5 |
| Agitan 350 - non-ionic surfactant | 0.5 |
| Dispex A40 - aqueous acrylic polymer | 0.5 |
| r-ITO - NIR absorber | 2.5 |
| 5,7-Dodecadiynedioic acid bis(octadecylamide) | 5.0 |
| Joncryl 8052 - aqueous acrylic binder | 37.5 |

(continued)

| Raw Material | pbw |
|---|---|
| Tyzor LA - aqueous titanium complex | 1.0 |
| Diethylene glycol - retarder | 5.0 |

The formulation was milled using a 50 ml Eiger-Torrance bead mill until a particle size <5 $\mu$m had been achieved. The ink was then coated onto both clear and white 50 $\mu$m OPP and PET films substrate at a coat weight of 10 gsm. The ink was also coated on to white label stock paper at 10 gsm.

The prepared substrates were coloured as follows:-

1. Using a dual wavelength laser system that emitted at both 1,550 nm and 266 nm.
2. A hot air gun (producing heat > 300°C) in combination with a UV germicidal lamp.

Each printed sample was exposed to heat and light as follows:

a. No heat, only UV light.
b. Heating followed the immediate exposure to UV light.
c. Heating, then allowing the sample to cool back to room temperature (30 minutes in the dark), then exposure to UV light.
d. Simultaneous heating and UV light.

It was seen that only the simultaneous heating and UB light exposure produced deep colours. The other three systems produced essentially no colour.

### Example 3

5,7-Dodecadiynedioic acid bis(octadecylamide) powder was added to PP and LDPE pellets at 0.25%. A few drops of rapeseed oil were added to assist dispersion and mixing. Closures were then prepared using an injection-moulding machine.

Activation was performed using a hot air gun (producing heat > 300°C) in combination with a UV germicidal lamp.

Each plastic part was exposed to heat and light as follows:

a. No heat, only UV light.
b. Heating, then allowing the sample to cool to room temperature then exposure to UV light.
c. Simultaneous heating and UV light.

It was seen that only the simultaneous heating and UV light exposure produced deep colours. The other two systems produced essentially no colour.

### Example 4

The following ink formulation was prepared. r-ITO (reduced indium tin oxide) is a NIR-absorber.

| Raw Material | pbw |
|---|---|
| Joncryl LMV7085 - aqueous styrene acrylic binder | 10.0 |
| Water | 32.5 |
| Dispelair CF49 - mineral oil anti-foam | 0.2 |
| Agitan 350 - non-ionic surfactant | 0.2 |
| Dispex A40 - aqueous acrylic polymer | 0.4 |
| r-ITO - NIR absorber | 2.0 |
| 5,7-dodecadiynedioic acid bis(octadecylamide) | 15.0 |

(continued)

| Raw Material | pbw |
|---|---|
| Joncryl 8052 - aqueous acrylic binder | 25.0 |
| Joncryl HPE 2157 - aqueous acrylic binder | 7.5 |
| Tyzor LA - aqueous titanium complex | 1.0 |
| Isopropanol - retarder | 2.5 |
| Tinuvin 1130 - UV absorber | 2.5 |
| Tinuvin 292 - hindered amine light stabiliser | 1.2 |

The ink formulation was applied to a clay-coated paper substrate using a flexographic printing technique at various coating weights including 10 $cm^3/m^2$ Anilox. The whiteness and transparency of the ink coating was good ($\Delta E<1$). Pre-lamination laser imaging performance was checked and found to be reasonable (optical density blackness [ODB] of 0.25 at fluence 4.04 $J/cm^2$; ODB>1 is desirable).

Subsequently, the coated substrate was subject to melt-extrusion lamination at a speed of 500 m/min with polyethylene (12 $g/m^2$) at 325°C. The effect of lamination on the background whiteness and transparency of the coating was assessed. The whiteness and transparency of the ink coating remained good ($\Delta E<1$). Activation during lamination was negligible.

Subsequently, a 20W, 1550 nm fibre laser fitted with a galvo mirror-based imaging head, linked to a PC, was used to expose the coated/laminated substrate at fluence 4.04 $J/cm^2$. This was followed by exposure to UV light at fluence 0.5 $J/cm^2$. An ODB of 0.28 could be achieved. ODM (optical density magenta) was significantly higher, up to 0.65. The activated colour is pink.

Further, a red ink test was used to determine the presence of laminate puncture damage caused by laser imaging. Thus, red ink was applied by a pipette to the laminated substrate subsequent to imaging. The application did not result in visible penetration of the red ink into the paperboard.

It may thus be concluded that substrates coated with an ink formulation of the invention may be covered with a thermoplastic polymer layer without discoloring the substrate. Further, the covered ink formulation may be marked without disrupting the outermost protecting polymer layer.

Whiteness and transparency were measured using a Gregtag MacBeth SpectroEye 5000 spectrophotometer (D65, 2°) according to the 1976 CIE (*L*, *a*, *b*) space, wherein $\Delta E = \sqrt{(L_2^* - L_1^*)^2 + (a_2^* - a_1^*)^2 + (b_2^* - b_1^*)^2}$ .

$\Delta E<1$ is typically not noticeable to the hypothetical standard observer.

**Examples 5 and 6**

7,9-Hexadecadiynedioic acid (ex. GFS Chemicals, 13.9 g, 0.05 mol) was dissolved in anhydrous, dry tetrahydrofuran (250 ml) under an atmosphere of nitrogen at 20°C. The solution was filtered under vacuum to remove any insoluble matter. Oxalyl chloride (25.4 g, 0.2 mol) was added followed by 5 drops of dimethylformamide. The reaction mixture was then left stirring for 20°C for 3 hours. The THF and excess oxalyl chloride were then removed by rotary evaporation. The oily acid chloride compound that had formed was re-dissolved in fresh dry, anhydrous THF (200 ml). To the THF solution was added either:

1. Dodecylamine (ex. Sigma-Aldrich) (20.4 g, 0.11 mol) and *N*-methyl morpholine (ex. Sigma-Aldrich) (12.9 g, 0.11 mol), dissolved in dry, anhydrous THF (100 ml); or
2. Octadecylamine (ex. Sigma-Aldrich) (29.6 g, 0.11 mol) and *N*-methyl morpholine (ex. Sigma-Aldrich) (12.9 g, 0.11 mol), dissolved in dry, anhydrous THF (100 ml).

The reaction mixtures were then left stirring at 20°C for 3 hours. After this time the resultant precipitate was collected by vacuum filtration, washed in THF (100 ml) and dried in a vacuum desiccator at 25°C.

Example 5. 7,9-Hexadecadiynedioic acid bis(dodecylamide); Yield = 25 g, -80%.

Example 6. 7,9-Hexadecadiynedioic acid bis(octadecylamide); Yield = 27.5 g, -70%.

**Example 7**

The following ink formulation was prepared

| Raw Material | pbw |
|---|---|
| Joncryl LMV7085 - aqueous styrene acrylic binder | 10.0 |
| Water | 32.5 |
| Dispelair CF49 - mineral oil anti-foam | 0.2 |
| Agitan 350 - non-ionic surfactant | 0.2 |
| Dispex A40 - aqueous acrylic polymer | 0.4 |
| r-ITO - NIR absorber | 2.0 |
| 7,9-Hexadecadiynedioic acid bis(octadecylamide) | 15.0 |
| Joncryl 8052 - aqueous acrylic binder | 25.0 |
| Joncryl HPE 2157 - aqueous acrylic binder | 7.5 |
| Tyzor LA - aqueous titanium complex | 1.0 |
| Isopropanol - retarder | 2.5 |
| Tinuvin 1130 - UV absorber | 2.5 |
| Tinuvin 292 - hindered amine light stabiliser | 1.2 |

**Example 8**

The following ink formulation was prepared

| Raw Material | pbw |
|---|---|
| Joncryl LMV7085 - aqueous styrene acrylic binder | 10.0 |
| Water | 32.5 |
| Dispelair CF49 - mineral oil anti-foam | 0.2 |
| Agitan 350 - non-ionic surfactant | 0.2 |
| Dispex A40 - aqueous acrylic polymer | 0.4 |
| r-ITO - NIR absorber | 2.0 |
| 7,9-Hexadecadiynedioic acid bis(dodecylamide) | 15.0 |
| Joncryl 8052 - aqueous acrylic binder | 25.0 |
| Joncryl HPE 2157 - aqueous acrylic binder | 7.5 |
| Tyzor LA - aqueous titanium complex | 1.0 |
| Isopropanol - retarder | 2.5 |
| Tinuvin 1130 - UV absorber | 2.5 |
| Tinuvin 292 - hindered amine light stabiliser | 1.2 |

The ink formulations prepared in Examples 7 and 8 were applied to a clay-coated paper substrate using a flexographic printing technique at various coating weights including 10 $cm^3/m^2$ Anilox. The whiteness and transparency of the ink coating was good ($\Delta E<1$). The coated substrates were imaged as described in Example 4. The laser imaging performance was found to be:

Example 7: Pre-lamination ODB = 0.64;     Post-lamination ODB = 0.80

(continued)

Example 8: Pre-lamination ODB = 0.37    Post-lamination ODB = 0.6

The magenta optical density (ODM) of the images post-lamination was also determined:

Example 7: Post-lamination ODM = 1.4

Example 8: Post-lamination ODM = 1.1

**Claims**

1. A method of forming a coloured substrate, comprising applying to or incorporating within the substrate a diacetylene compound, and exposing the substrate to (i) a first, activating stimulus that converts the diacetylene compound from an unreactive to UV light to a reactive form, and (ii) a second stimulus that causes the reactive form of the diacetylene compound to polymerize and form the coloured substrate, wherein the diacetylene compound reverts to its unreactive form on removal of the activating stimulus and is of formula II:

$$CH_3\text{-}(CH_2)_y\text{-}NH\text{-}CO\text{-}(CH_2)_z\text{-}C\equiv C\text{-}C\equiv C\text{-}(CH_2)_z\text{-}CO\text{-}NH\text{-}(CH_2)_y\text{-}CH_3 \qquad \text{(Formula II)}$$

wherein:

each y is 9, 11, 13, 15 or 17; and
each z is 3 or 5;

optionally wherein the second stimulus is light having a wavelength in the range 200 nm to 25 $\mu$m, optionally wherein the activating stimulus is selected from heat, light having a wavelength in the range 100 nm to 20,000 nm, electric or magnetic fields, chemical agents and biological agents and optionally wherein the activating stimulus and the second stimulus are provided by the same light source.

2. A method according to claim 1, wherein the substrate comprises a near-infrared-absorbing agent which absorbs light having a wavelength in the range 700 nm to 2500 nm, optionally wherein the near-infrared-absorbing agent is selected from organic dyes/pigments, conductive polymers, inorganic copper (II) salts and non-stoichiometric inorganic compounds and optionally wherein the diacetylene compound and the near-infrared-absorbing agent are applied to the substrate in the same coating, in separate coatings, or are embedded in the substrate.

3. A method according to any preceding claim, wherein the substrate or a coating thereon comprises a further colour-change agent, preferably a metal oxyanion compound, charrable agent, leuco dye or charge-transfer agent, wherein the further colour-change agent is ammonium octamolybdate or sodium metaborate or a charrable polysaccharide, wherein the polysaccharide is optionally used in combination with a metal salt.

4. A method according to any preceding claim, wherein the substrate is a plastic material.

5. A method according to any preceding claim, wherein the substrate is primary or secondary packaging, optionally wherein the substrate is of the type used to make sterilised food packaging containers, optionally wherein the substrate includes paper, paperboard and/or cardboard, optionally wherein the substrate is laminated, optionally wherein the substrate is laminated clay-coated paper and optionally wherein the substrate displays human-readable and/or machine-readable information.

6. A coloured substrate obtainable by a method according to any preceding claim.

7. A compound of the formula II:

$$CH_3\text{-}(CH_2)_y\text{-}NH\text{-}CO\text{-}(CH_2)_z\text{-}C\equiv C\text{-}C\equiv C\text{-}(CH_2)_z\text{-}CO\text{-}NH\text{-}(CH_2)_y\text{-}CH_3 \qquad \text{(II)}$$

wherein:

each y is 9, 11, 13, 15 or 17; and
each z is 3 or 5;
optionally wherein the compound is 5,7-dodecadiynedioic acid bis(octadecylamide), 7,9-hexadecadiynedioic acid bis(dodecylamide) or 7,9-hexadecadiynedioic acid bis(octadecylamide).

## Patentansprüche

1. Ein Verfahren zum Bilden eines farbigen Substrats, das Folgendes umfasst, nämlich das Auftragen oder Inkorporieren einer Diacetylenverbindung auf oder in das Substrat und Aussetzen des Substrats an (i) ein erstes aktivierendes Stimulans, das die Diacetylenverbindung von einer gegenüber UV-Licht nicht reaktiven Form in eine reaktive Form umsetzt, und (ii) ein zweites Stimulans, das verursacht, dass die reaktive Form der Diacetylenverbindung polymerisiert und das farbige Substrat bildet, wobei die Diacetylenverbindung nach Entfernen des aktivierenden Stimulans wieder zu ihrer nicht reaktiven Form zurückkehrt und folgende Formel II aufweist:

$$CH_3\text{-}(CH_2)_y\text{-}NH\text{-}CO\text{-}(CH_2)_z\text{-}C{\equiv}C\text{-}C{\equiv}C\text{-}(CH_2)_z\text{-}CO\text{-}NH\text{-}(CH_2)_y\text{-}CH_3 \qquad \text{(Formel II)}$$

wobei:

jedes y 9, 11, 13, 15 oder 17 ist; und
jedes z 3 oder 5 ist;

wobei optional das zweite Stimulans Licht ist, das eine Wellenlänge im Bereich von 200 nm bis 25 $\mu$m aufweist, wobei optional das aktivierende Stimulans aus Wärme, Licht mit einer Wellenlänge im Bereich von 100 nm bis 20.000 nm, elektrischen oder magnetischen Feldern, chemischen Wirkstoffen und biologischen Wirkstoffen ausgewählt wird und wobei optional das aktivierende Stimulans und and zweite Stimulans von der selben Lichtquelle bereitgestellt werden.

2. Ein Verfahren entsprechend Anspruch 1, wobei das Substrat ein nahinfrarotabsorbierendes Mittel umfasst, das Licht mit einer Wellenlänge im Bereich von 700 nm bis 2500 nm absorbiert, wobei das nahinfrarotabsorbierende Mittel optional aus organischen Farbstoffen/Pigmenten, leitfähigen Polymeren, anorganischen Kupfer-(II)-Salzen und nicht stöchiometrischen anorganischen Verbindungen ausgewählt wird und wobei die Diacetylenverbindung und das nahinfrarotabsorbierende Mittel optional in der selben Schicht, in separaten Schichten auf das Substrat aufgetragen oder in das Substrat integriert werden.

3. Ein Verfahren entsprechend einem der vorhergehenden Ansprüche, wobei das Substrat oder eine Beschichtung auf diesem ein weiteres farbveränderndes Mittel umfasst, bevorzugt eine Metall-Oxyanionen-Verbindung, ein verkohlbares Mittel, ein Leukofarbstoff oder ein ladungsübertragendes Mittel, wobei das weitere farbverändernde Mittel Ammonium-Oktamolybdat oder Natriummetaborat oder ein verkohlbares Polysaccharid ist, wobei das Polysaccharid optional in Kombination mit einem Metallsalz verwendet wird.

4. Ein Verfahren entsprechend einem der vorhergehenden Ansprüche, wobei das Substrat ein Plastikmaterial ist.

5. Ein Verfahren entsprechend einem der vorhergehenden Ansprüche, wobei das Substrat primäre oder sekundäre Verpackung ist, wobei das Substrat optional von der Art ist, wie sie zur Herstellung sterilisierter Lebensmittelverpackungsbehälter verwendet wird, wobei das Substrat optional Papier, Pappe und/oder Karton miteinschließt, wobei das Substrat optional laminiert ist, wobei das Substrat optional laminiertes, kaolinbeschichtetes Papier ist und wobei das Substrat optional für Menschen lesbare und/oder maschinenlesbare Information anzeigt.

6. Ein farbiges Substrat, das durch ein Verfahren entsprechend einem der vorgehenden Ansprüche erhalten werden kann.

7. Eine Verbindung mit der Formel II:

$$CH_3\text{-}(CH_2)_y\text{-}NH\text{-}CO\text{-}(CH_2)_z\text{-}C{\equiv}C\text{-}C{\equiv}C\text{-}(CH_2)_z\text{-}CO\text{-}NH\text{-}(CH_2)_y\text{-}CH_3 \qquad \text{(II)}$$

wobei:

jedes y 9, 11, 13, 15 oder 17 ist; und
jedes z 3 oder 5 ist;

wobei die Verbindung optional 5,7-Dodecadiensäure-Bis(octadecylamid), 7,9-Hexadecadiensäure-Bis(dodecylamid) oder 7,9-Hexadecadiensäure-Bis(octadecylamid) ist.

## Revendications

1. Procédé de formation d'un substrat coloré comprenant l'application sur ou l'incorporation dans le substrat d'un composé diacétylénique et l'exposition du substrat (i) à un premier stimulus d'activation qui convertit le composé diacétylénique d'une forme non réactive à la lumière UV en une forme réactive et (ii) à un deuxième stimulus qui conduit la forme réactive du composé diacétylénique à polymériser et à former le substrat coloré, le composé diacétylénique retournant à sa forme non réactive au retrait du stimulus d'activation et répondant à la formule II :

$$CH_3\text{-}(CH_2)_y\text{-}NH\text{-}CO\text{-}(CH_2)_z\text{-}C{\equiv}C\text{-}C{\equiv}C\text{-}(CH_2)_z\text{-}CO\text{-}NH\text{-}(CH_2)_y\text{-}CH_3 \qquad \text{(Formule II)}$$

où :

chaque y prend la valeur 9, 11, 13, 15 ou 17 ; et
chaque z prend la valeur 3 ou 5 ;

éventuellement où le deuxième stimulus est une lumière ayant une longueur d'onde dans la plage de 200 nm à 25 $\mu$m, éventuellement où le stimulus d'activation est sélectionné parmi la chaleur, une lumière ayant une longueur d'onde dans la plage de 100 nm à 20 000 nm, des champs électriques ou magnétiques, des agents chimiques et des agents biologiques et éventuellement où le stimulus d'activation et le deuxième stimulus sont fournis par la même source lumineuse.

2. Procédé selon la revendication 1, où le substrat comprend un agent absorbant le proche infrarouge qui absorbe une lumière ayant une longueur d'onde dans la plage de 700 nm à 2 500 nm, éventuellement où l'agent absorbant le proche infrarouge est sélectionné parmi des colorants/pigments organiques, des polymères conducteurs, des sels de cuivre (II) inorganiques et des composés inorganiques non stoechiométriques, et éventuellement où le composé diacétylénique et l'agent absorbant le proche infrarouge sont appliqués sur le substrat dans le même revêtement ou dans des revêtements séparés ou sont incorporés dans le substrat.

3. Procédé selon l'une quelconque des revendications précédentes, où le substrat ou un revêtement que celui-ci comporte comprend un agent de changement de la couleur supplémentaire, de préférence un composé à oxyanion métallique, un agent carbonisable, un colorant leuco ou un agent de transfert de charge, l'agent de changement de la couleur supplémentaire étant l'octamolybdate d'ammonium ou le métaborate de sodium ou un polysaccharide carbonisable, où le polysaccharide est éventuellement utilisé en combinaison avec un sel métallique.

4. Procédé selon l'une quelconque des revendications précédentes, où le substrat est une matière plastique.

5. Procédé selon l'une quelconque des revendications précédentes où le substrat est un emballage primaire ou secondaire, éventuellement où le substrat est du type utilisé pour fabriquer des récipients d'emballage alimentaire stérilisés, éventuellement où le substrat inclut un papier, un papier cartonné et/ou un carton, éventuellement où le substrat est stratifié, éventuellement où le substrat est un papier couché au kaolin laminé et éventuellement où le substrat porte une information lisible par l'homme et/ou une information lisible par machine.

6. Substrat coloré qui peut être obtenu par un procédé selon l'une quelconque des revendications précédentes.

7. Composé de formule II :

$$CH_3\text{-}(CH_2)_y\text{-}NH\text{-}CO\text{-}(CH_2)_z\text{-}C{\equiv}C\text{-}C{\equiv}C\text{-}(CH_2)_,\text{-}CO\text{-}NH\text{-}(CH_2)_y\text{-}CH_3 \qquad \text{(II)}$$

où :

chaque y prend la valeur 9, 11, 13, 15 ou 17 ; et

chaque z prend la valeur 3 ou 5 ;
éventuellement où le composé est le bis(octadécylamide) de l'acide 5,7-dodécadiynedioïque, le bis(dodécylamide) de l'acide 7,9-hexadécadiynedioïque ou le bis(octadécylamide) de l'acide 7,9-hexadécadiynedioïque.

Figure 1

10

18          18

13

12

11

16

15

17

14

Figure 2

30

18

32

*Fig 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010042001 A
- WO 2011035909 A
- WO 2006018640 A
- US 6911262 B
- WO 2008050153 A
- WO 200512442 A
- WO 2005068207 A
- WO 2005095516 A
- WO 2006129086 A
- WO 2007045912 A
- WO 2002068205 A
- WO 2006129078 A
- WO 2004043704 A
- WO 2002074548 A
- WO 2007063339 A
- WO 2006051309 A
- WO 2009010393 A

**Non-patent literature cited in the description**

- Developments in the Chemistry and Technology of Organic dyes. Blackwell Scientific, 1984
- Infrared Absorbing Dyes. Plenum Press, 1990